# EUROPEAN PATENT APPLICATION

(11) **EP 2 664 624 A1**
(43) Date of publication of application: **20.11.2013**
(21) Application number: 12168095.3
(22) Date of filing: 15.05.2012
(51) Int. Cl.: C07K 14/415, A61K 39/35, C07K 16/16

(54) **Allergen variants**

(71) Applicant: BIOMAY AG, 1090 Wien (AT)
(72) Inventor: Breiteneder, Heimo, 1070 Vienna (AT); Bublin, Merima, 1150 Vienna (AT); Radauer, Christian, 1150 Vienna (AT)
(74) Representative: KLIMENT & HENHAPEL

(57) **Abstract**

The present invention relates to a method for producing a hypoallergenic variant of an allergenic polypeptide having one or more loop regions comprising the step of modifying at least one loop region within the allergenic polypeptide by dividing said at least one loop region into at least two fragments, each of said fragments comprising at least 4 amino acid residues and rejoining said at least two fragments in a different order than in said allergenic polypeptide.

SEQUENCE LISTING

<110>  Biomay AG

<120>  Allergen Variants

<130>  R 60467

<160>  9

<170>  PatentIn version 3.5

<210>  1
<211>  522
<212>  PRT
<213>  Artificial Sequence

<220>
<223>  Allergen variant

<400>  1

Gly Arg Trp Gly Pro Ala Gly Pro Arg Gln Pro Arg Arg Glu Glu Gly
1               5                   10                  15

Trp Arg Gln Pro Arg Glu Asp Trp Arg Glu Arg Glu Arg Glu Glu Asp
            20                  25                  30

Glu Gly Glu Gln Glu Trp Gly Thr Arg Lys Ile Arg Pro Glu Gly Arg
        35                  40                  45

Arg Arg Pro Ser His Gln Gln Pro Pro Gly Ser His Val Arg Glu Glu
    50                  55                  60

Thr Ser Arg Asn Asn Pro Phe Tyr Phe Pro Ser Arg Arg Phe Ser Thr
65                  70                  75                  80

Arg Tyr Gly Asn Gln Asn Gly Arg Ile Arg Val Leu Gln Gln Phe Gln
                85                  90                  95

Asn Leu Gln Asn Arg Phe Asp Gln Arg Ser Arg His Arg Ile Val Gln
            100                 105                 110

Ile Glu Ala Lys Pro Asn Thr Leu Val Leu Pro Lys His Ala Asp Ala
        115                 120                 125

Asp Asn Ile Leu Val Ile Gln Gln Gly Gln Ala Thr Val Thr Val Ala
    130                 135                 140

Asn Gly Asn Asn Arg Lys Ser Phe Asn Leu Asp Glu Gly His Ala Leu
145                 150                 155                 160

Arg Ile Pro Ser Gly Phe Ile Ser Tyr Ile Leu Asn Arg His Asp Asn
                165                 170                 175

Gln Asn Leu Arg Val Ala Lys Ile Ser Met Pro Val Asn Thr Pro Gly
            180                 185                 190

Gln Phe Glu Asp Phe Phe Pro Ala Ser Ser Arg Asp Gln Ser Ser Tyr
        195                 200                 205

Leu Glu Ala Ala Phe Asn Ala Glu Phe Gln Gly Phe Ser Arg Asn Thr
    210                 215                 220

Leu Glu Glu Arg Gly Gln Arg Arg Trp Glu Glu Asn Ala Gly Gly Glu
225                 230                 235                 240

Gln Asn Glu Ile Arg Arg Val Leu Leu Ser Thr Arg Ser Ser Glu Asn
                245                 250                 255

Asn Glu Gly Val Ile Val Lys Val Ser Lys Glu His Val Glu Glu Val
            260                 265                 270

Ser Lys Lys Gly Ser Glu Leu Thr Lys His Ala Lys Ser Glu Glu Gly
        275                 280                 285

Asp Ile Thr Asn Pro Ile Asn Leu Arg Glu Gly Glu Pro Asp Leu Ser
    290                 295                 300

Asn Asn Phe Gly Lys Leu Phe Glu Val Lys Pro Asp Lys Lys Asn Pro
305                 310                 315                 320

Gln Leu Gln Asp Leu Asp Met Met Leu Thr Cys Val Glu Ile Lys Glu
                325                 330                 335

Gly Ala Leu Met Leu Pro His Phe Asn Ser Lys Ala Met Val Ile Val
            340                 345                 350

Val Val Asn Lys Gly Thr Gly Asn Leu Glu Leu Val Ala Val Arg Lys
        355                 360                 365

Glu Glu Glu Glu Glu Gly Ser Asn Glu Glu Glu Glu Asp Glu Asp Gln
    370                 375                 380

Gln Gln Arg Gly Arg Arg Arg Glu Val Arg Arg Tyr Thr Ala Arg Leu
385                 390                 395                 400

Lys Glu Gly Asp Val Phe Ile Met Pro Ala Ala His Pro Val Ala Ile
                405                 410                 415

Asn Ala Ser Ser Glu Leu His Leu Leu Gly Phe Gly Ile Asn Ala Glu
            420                 425                 430

Asn Asn His Arg Ile Phe Leu Ala Gly Asp Lys Asp Asn Val Ile Ser
        435                 440                 445

Gly Glu Gln Val Glu Lys Lys Asp Leu Ala Phe Pro Gly Asp Gln Ile
    450                 455                 460

Glu Lys Gln Ala Leu Ile Lys Asn Gln Lys Glu Ser His Phe Val Ser
465                 470                 475                 480

Ala Arg Ser Ser Pro Glu Lys Glu Ser Pro Pro Gln Ser Gln Ser Gln
                485                 490                 495

Ser Pro Leu Ser Ile Leu Lys Ala Phe Asn Asn Gln Gly Gly Lys Gly
            500                 505                 510

Pro Leu Glu Lys Glu Asp Gln Glu Glu Glu
        515                 520

<210>  2
<211>  151
<212>  PRT
<213>  Artificial Sequence

<220>
<223>  Allergen variant

<400>  2

Leu Gln Gly Asp Arg Arg Gln Gln Trp Glu Arg Cys Gln Ser Gln Leu
1               5                   10                  15

Glu Arg Ala Asn Leu Arg Pro Cys Glu Gln His Leu Met Gln Lys Ile
            20                  25                  30

Gln Arg Ser Pro Ser Gln Asp Pro Tyr Ser Pro Ser Asp Glu Asp Ser
        35                  40                  45

Tyr Gly Arg Asp Pro Tyr Pro Ser Pro Tyr Asp Arg Arg Gly Ala Gln
    50                  55                  60

Asp Pro Asp Arg Arg Asp Pro Tyr Ser Gly Ser Ser Gln His Gln Glu
65                  70                  75                  80

Arg Cys Cys Asn Glu Leu Asn Glu Phe Glu Asn Asn Gln Arg Cys Met
                85                  90                  95

Cys Glu Ala Leu Gln Gln Ile Met Glu Asn Gln Ser Asp Arg Leu Gln
            100                 105                 110

Gly Arg Gln Gln Glu Gln Gln Phe Lys Arg Glu Leu Arg Asn Leu Pro
        115                 120                 125

Gln Gln Cys Gly Leu Glu Val Glu Ser Arg Cys Asp Leu Arg Ala Pro
    130                 135                 140

Gln Gly Gly Arg Asp Arg Tyr
145                 150

<210>  3
<211>  151
<212>  PRT
<213>  Artificial Sequence

<220>
<223>  Allergen variant

<400>  3

Leu Gln Gly Asp Arg Arg Gln Gln Trp Glu Arg Cys Gln Ser Gln Leu
1               5                   10                  15

Glu Arg Ala Asn Leu Arg Pro Cys Glu Gln His Leu Met Gln Lys Ile
            20                  25                  30

Gln Arg Arg Asp Pro Tyr Ser Pro Asp Glu Asp Ser Tyr Gly Ser Pro
        35                  40                  45

Ser Gln Asp Ser Gln Asp Pro Tyr Pro Asp Arg Arg Asp Pro Tyr Asp
    50                  55                  60

Arg Arg Gly Ala Tyr Ser Pro Ser Pro Gly Ser Ser Gln His Gln Glu
65                  70                  75                  80

Arg Cys Cys Asn Glu Leu Asn Glu Phe Glu Asn Asn Gln Arg Cys Met
                85                  90                  95

Cys Glu Ala Leu Gln Gln Ile Met Glu Asn Gln Gly Arg Gln Leu Gln
            100                 105                 110

Ser Asp Arg Gln Glu Gln Gln Phe Lys Arg Glu Leu Arg Asn Leu Pro
        115                 120                 125

Gln Gln Cys Gly Leu Glu Val Glu Ser Arg Cys Asp Leu Arg Ala Pro
    130                 135                 140

Gln Gly Gly Arg Asp Arg Tyr
145                 150

<210>  4
<211>  518
<212>  PRT
<213>  Artificial Sequence

<220>

<223>  Allergen variant

<400>  4

Ile Ser Phe Gln Arg Leu Asn Ala Gln Arg Pro Phe Arg Gln Gln Pro
1               5                   10                  15

Glu Glu Asn Ala Cys Gln Asp Asn Arg Ile Glu Ser Glu Gly Gly Tyr
            20                  25                  30

Ile Glu Thr Trp Phe Glu Cys Ala Gly Asn Pro Asn Asn Gln Glu Val
        35                  40                  45

Ala Leu Ser Arg Leu Val Leu Arg Arg Asn Ala Leu Arg Arg Pro Phe
    50                  55                  60

Tyr Ser Asn Ala Pro Gln Glu Ile Phe Ile Gln Gln Gly Arg Gly Tyr
65                  70                  75                  80

Phe Gly Leu Ile Phe Pro Gly Cys Pro Ser Arg Arg His Gln Ser Gln
                85                  90                  95

Arg Pro Pro Thr Tyr Glu Glu Pro Ala Gln Gln Gly Gln Gln Gln Gln
            100                 105                 110

Asp Ser His Gln Lys Arg Arg Phe Gln Gly Gln Asp Gln Ser Val His
        115                 120                 125

Arg Phe Asp Glu Gly Asp Leu Ile Ala Val Pro Thr Gly Val Ala Phe
    130                 135                 140

Trp Met Tyr Asn Asp His Asp Thr Asp Val Val Ala Val Ser Leu Thr
145                 150                 155                 160

Asp Leu Asp Gln Phe Pro Arg Thr Asn Asn Asn Asp Asn Gln Arg Phe
                165                 170                 175

Asn Leu Leu Arg Tyr Gln Gln Gln Ser Arg Ala Gly Asn His Glu Gln
            180                 185                 190

Glu Phe Tyr Ser Pro Gln Thr Gln Pro Lys Arg Arg Ser Leu Pro Tyr
        195                 200                 205

Ser Pro Arg Gly Gln His Gly Arg Arg Glu Gln Glu Asp Arg Glu Phe
    210                 215                 220

Ser Pro Glu Gly Gly Asn Ile Phe Ser Gly Arg Ala Gly Gln Glu Gln
225                 230                 235                 240

Glu Asn Ala Phe Gln Val Asp Asp Arg Gln Phe Thr Pro Glu Phe Leu

245                 250                 255

Ala Gln Asn Glu Ser Asp Glu Gln Gly Ile Leu Gln Asn Leu Arg Gly
            260                 265                 270

Glu Ala Ile Val Thr Val Pro Asp Arg Lys Arg Arg Gln Gln Arg Gly
        275                 280                 285

Gly Leu Arg Ile Leu Ser Glu Tyr Asp Glu Asp Glu Tyr Glu Tyr Glu
    290                 295                 300

Arg Pro Asp Glu Glu Glu Tyr Asp Glu Glu Glu Arg Gln Gln Arg Gly
305                 310                 315                 320

Ser Gly Asn Gly Ile Glu Asp Arg Arg Arg Gly Arg Gly Ser Glu Thr
                325                 330                 335

Ile Cys Thr Ala Ser Phe Lys Lys Asn Ile Gly Arg Asn Arg Ser Pro
            340                 345                 350

Asp Ile Tyr Asn Pro Gln Ala Gly Ser Leu Lys Thr Ala Asn Ile Leu
        355                 360                 365

Arg Trp Leu Gly Glu Leu Gln Leu Asn Leu Leu Leu Ser Ala Glu Tyr
    370                 375                 380

Gly Asn Leu Tyr Arg Asn Ala Leu Phe Val Pro His Tyr Asn Thr Asn
385                 390                 395                 400

Ala His Ser Ile Ile Tyr Ala Leu Arg Gly Arg Ala His Val Gln Val
                405                 410                 415

Val Asp Ser Asn Gly Asp Arg Val Phe Asp Glu Glu Leu Gln Glu Gly
            420                 425                 430

His Val Leu Val Val Pro Gln Asn Phe Ala Val Ala Gly Lys Ser Gln
        435                 440                 445

Ser Glu Asn Phe Glu Tyr Val Ala Phe Lys Thr Asp Ser Arg Pro Ser
    450                 455                 460

Ile Ala Asn Leu Asp Asn Leu Pro Glu Glu Val Ala Gly Glu Asn Ser
465                 470                 475                 480

Phe Ile Val Ala Asn Ser Tyr Gly Leu Leu Lys Asn Asn Asn Pro Phe
                485                 490                 495

Lys Pro Arg Glu Gln Ala Arg Gln Phe Phe Val Pro Pro Ser Glu Gln
            500                 505                 510

Ser Leu Arg Ala Val Ala
        515

<210>  5
<211>  124
<212>  PRT
<213>  Artificial Sequence

<220>
<223>  Allergen variant

<400>  5

Met Arg Gln Gly Asp Ser Ser Arg Arg Glu Arg Gly Ser Cys Glu Arg
1               5                   10                  15

Gln Val Asp Arg Val Asn Leu Lys Pro Cys Glu Gln His Ile Met Gln
            20                  25                  30

Arg Ile Met Gly Ser Tyr Asp Ile Arg Ser Thr Glu Gln Glu Gln Tyr
        35                  40                  45

Asp Arg Ser Ser Asp Gln Gln Gln Arg Cys Cys Asp Glu Leu Asn Glu
    50                  55                  60

Met Glu Asn Thr Gln Arg Cys Met Cys Glu Ala Leu Gln Gln Ile Met
65                  70                  75                  80

Glu Asn Gln Cys Asp Arg Leu Gln Asp Arg Gln Met Val Gln Gln Phe
                85                  90                  95

Lys Arg Glu Leu Met Asn Leu Pro Gln Gln Cys Asn Leu Asp Val Ser
            100                 105                 110

Gly Arg Cys Asp Phe Arg Ala Pro Gln Gly Arg Cys
        115                 120

<210>  6
<211>  522
<212>  PRT
<213>  Arachis hypogaea

<400>  6

Arg Gln Pro Arg Arg Glu Glu Gly Gly Arg Trp Gly Pro Ala Gly Pro
1               5                   10                  15

Arg Glu Arg Glu Arg Glu Glu Asp Trp Arg Gln Pro Arg Glu Asp Trp
            20                  25                  30

Arg Arg Pro Ser His Gln Gln Pro Arg Lys Ile Arg Pro Glu Gly Arg
        35                  40                  45

Glu Gly Glu Gln Glu Trp Gly Thr Pro Gly Ser His Val Arg Glu Glu
    50                  55                  60

Thr Ser Arg Asn Asn Pro Phe Tyr Phe Pro Ser Arg Arg Phe Ser Thr
65                  70                  75                  80

Arg Tyr Gly Asn Gln Asn Gly Arg Ile Arg Val Leu Gln Arg Phe Asp
                85                  90                  95

Gln Arg Ser Arg Gln Phe Gln Asn Leu Gln Asn His Arg Ile Val Gln
            100                 105                 110

Ile Glu Ala Lys Pro Asn Thr Leu Val Leu Pro Lys His Ala Asp Ala
        115                 120                 125

Asp Asn Ile Leu Val Ile Gln Gln Gly Gln Ala Thr Val Thr Val Ala
    130                 135                 140

Asn Gly Asn Asn Arg Lys Ser Phe Asn Leu Asp Glu Gly His Ala Leu
145                 150                 155                 160

Arg Ile Pro Ser Gly Phe Ile Ser Tyr Ile Leu Asn Arg His Asp Asn
                165                 170                 175

Gln Asn Leu Arg Val Ala Lys Ile Ser Met Pro Val Asn Thr Pro Gly
            180                 185                 190

Gln Phe Glu Asp Phe Phe Pro Ala Ser Ser Arg Asp Gln Ser Ser Tyr
        195                 200                 205

Leu Gln Gly Phe Ser Arg Asn Thr Leu Glu Ala Ala Phe Asn Ala Glu
    210                 215                 220

Phe Asn Glu Ile Arg Arg Val Leu Leu Glu Glu Asn Ala Gly Gly Glu
225                 230                 235                 240

Gln Glu Glu Arg Gly Gln Arg Arg Trp Ser Thr Arg Ser Ser Glu Asn
                245                 250                 255

Asn Glu Gly Val Ile Val Lys Val Ser Lys Glu His Val Glu Glu Leu
            260                 265                 270

Thr Lys His Ala Lys Ser Val Ser Lys Lys Gly Ser Glu Glu Glu Gly
        275                 280                 285

Asp Ile Thr Asn Pro Ile Asn Leu Arg Glu Gly Glu Pro Asp Leu Ser
    290                 295                 300

Asn Asn Phe Gly Lys Leu Phe Glu Val Lys Pro Asp Lys Lys Asn Pro
305                 310                 315                 320

Gln Leu Gln Asp Leu Asp Met Met Leu Thr Cys Val Glu Ile Lys Glu
                325                 330                 335

Gly Ala Leu Met Leu Pro His Phe Asn Ser Lys Ala Met Val Ile Val
            340                 345                 350

Val Val Asn Lys Gly Thr Gly Asn Leu Glu Leu Val Ala Val Arg Lys
        355                 360                 365

Glu Gln Gln Gln Arg Gly Arg Arg Glu Glu Glu Glu Asp Glu Asp Glu
    370                 375                 380

Glu Glu Glu Gly Ser Asn Arg Glu Val Arg Arg Tyr Thr Ala Arg Leu
385                 390                 395                 400

Lys Glu Gly Asp Val Phe Ile Met Pro Ala Ala His Pro Val Ala Ile
                405                 410                 415

Asn Ala Ser Ser Glu Leu His Leu Leu Gly Phe Gly Ile Asn Ala Glu
            420                 425                 430

Asn Asn His Arg Ile Phe Leu Ala Gly Asp Lys Asp Asn Val Ile Asp
        435                 440                 445

Gln Ile Glu Lys Gln Ala Lys Asp Leu Ala Phe Pro Gly Ser Gly Glu
    450                 455                 460

Gln Val Glu Lys Leu Ile Lys Asn Gln Lys Glu Ser His Phe Val Ser
465                 470                 475                 480

Ala Arg Pro Gln Ser Gln Ser Gln Ser Pro Ser Ser Pro Glu Lys Glu
                485                 490                 495

Ser Pro Glu Lys Glu Asp Gln Glu Glu Glu Asn Gln Gly Gly Lys Gly
            500                 505                 510

Pro Leu Leu Ser Ile Leu Lys Ala Phe Asn
        515                 520

<210>  7
<211>  151
<212>  PRT
<213>  Arachis hypogaea

<400>  7

Arg Gln Gln Trp Glu Leu Gln Gly Asp Arg Arg Cys Gln Ser Gln Leu

1               5                   10                  15

Glu Arg Ala Asn Leu Arg Pro Cys Glu Gln His Leu Met Gln Lys Ile
            20                  25                  30

Gln Arg Asp Glu Asp Ser Tyr Gly Arg Asp Pro Tyr Ser Pro Ser Gln
        35                  40                  45

Asp Pro Tyr Ser Pro Ser Gln Asp Pro Asp Arg Arg Asp Pro Tyr Ser
    50                  55                  60

Pro Ser Pro Tyr Asp Arg Arg Gly Ala Gly Ser Ser Gln His Gln Glu
65                  70                  75                  80

Arg Cys Cys Asn Glu Leu Asn Glu Phe Glu Asn Asn Gln Arg Cys Met
                85                  90                  95

Cys Glu Ala Leu Gln Gln Ile Met Glu Asn Gln Ser Asp Arg Leu Gln
            100                 105                 110

Gly Arg Gln Gln Glu Gln Gln Phe Lys Arg Glu Leu Arg Asn Leu Pro
        115                 120                 125

Gln Gln Cys Gly Leu Arg Ala Pro Gln Arg Cys Asp Leu Glu Val Glu
    130                 135                 140

Ser Gly Gly Arg Asp Arg Tyr
145                 150

<210>  8
<211>  518
<212>  PRT
<213>  Arachis hypogaea

<400>  8
   Ile Ser Phe Gln Arg Leu Asn Ala Gln Arg Pro Phe Arg Gln Gln Pro
   1               5                   10                  15
   Glu Glu Asn Ala Cys Gln Asp Asn Arg Ile Glu Ser Glu Gly Gly Tyr
               20                  25                  30
   Ile Glu Thr Trp Phe Glu Cys Ala Gly Asn Pro Asn Asn Gln Glu Val
           35                  40                  45
   Ala Leu Ser Arg Leu Val Leu Arg Arg Asn Ala Leu Arg Arg Pro Phe
       50                  55                  60
   Tyr Ser Asn Ala Pro Gln Glu Ile Phe Ile Gln Gln Gly Arg Gly Tyr
   65                  70                  75                  80
   Phe Gly Leu Ile Phe Pro Gly Cys Pro Ser Thr Tyr Glu Glu Pro Ala
                   85                  90                  95
   Gln Gln Gly Arg Arg His Gln Ser Gln Arg Pro Pro Arg Arg Phe Gln
               100                 105                 110
   Gly Gln Asp Gln Ser Gln Gln Gln Gln Asp Ser His Gln Lys Val His
           115                 120                 125
   Arg Phe Asp Glu Gly Asp Leu Ile Ala Val Pro Thr Gly Val Ala Phe
       130                 135                 140
   Trp Met Tyr Asn Asp His Asp Thr Asp Val Val Ala Val Ser Leu Thr
   145                 150                 155                 160
   Asp Thr Asn Asn Asn Asp Asn Gln Leu Asp Gln Phe Pro Arg Arg Phe
                   165                 170                 175
   Asn Leu Ala Gly Asn His Glu Gln Glu Phe Leu Arg Tyr Gln Gln Gln
               180                 185                 190
   Ser Arg Arg Arg Ser Leu Pro Tyr Ser Pro Tyr Ser Pro Gln Thr Gln
           195                 200                 205
   Pro Lys Gln Glu Asp Arg Glu Phe Ser Pro Arg Gly Gln His Gly Arg
       210                 215                 220
   Arg Glu Arg Ala Gly Gln Glu Gln Glu Asn Glu Gly Gly Asn Ile Phe
   225                 230                 235                 240
   Ser Gly Phe Thr Pro Glu Phe Leu Ala Gln Ala Phe Gln Val Asp Asp
                   245                 250                 255
   Arg Gln Ile Leu Gln Asn Leu Arg Gly Glu Asn Glu Ser Asp Glu Gln
               260                 265                 270
   Gly Ala Ile Val Thr Val Arg Gly Gly Leu Arg Ile Leu Ser Pro Asp
           275                 280                 285
   Arg Lys Arg Arg Gln Gln Tyr Glu Arg Pro Asp Glu Glu Glu Glu Tyr
       290                 295                 300
   Asp Glu Asp Glu Tyr Glu Tyr Asp Glu Glu Glu Arg Gln Gln Asp Arg
   305                 310                 315                 320
   Arg Arg Gly Arg Gly Ser Arg Gly Ser Gly Asn Gly Ile Glu Glu Thr
                   325                 330                 335
   Ile Cys Thr Ala Ser Phe Lys Lys Asn Ile Gly Arg Asn Arg Ser Pro
               340                 345                 350
   Asp Ile Tyr Asn Pro Gln Ala Gly Ser Leu Lys Thr Ala Asn Glu Leu
           355                 360                 365
   Gln Leu Asn Leu Leu Ile Leu Arg Trp Leu Gly Leu Ser Ala Glu Tyr
       370                 375                 380
   Gly Asn Leu Tyr Arg Asn Ala Leu Phe Val Pro His Tyr Asn Thr Asn
   385                 390                 395                 400
   Ala His Ser Ile Ile Tyr Ala Leu Arg Gly Arg Ala His Val Gln Val
                   405                 410                 415
   Val Asp Ser Asn Gly Asp Arg Val Phe Asp Glu Glu Leu Gln Glu Gly
               420                 425                 430
   His Val Leu Val Val Pro Gln Asn Phe Ala Val Ala Gly Lys Ser Gln
           435                 440                 445
   Ser Glu Asn Phe Glu Tyr Val Ala Phe Lys Thr Asp Ser Arg Pro Ser
       450                 455                 460
   Ile Ala Asn Leu Ala Gly Glu Asn Ser Phe Ile Asp Asn Leu Pro Glu
   465                 470                 475                 480
   Glu Val Val Ala Asn Ser Tyr Gly Leu Pro Arg Glu Gln Ala Arg Gln
                   485                 490                 495
   Leu Lys Asn Asn Asn Pro Phe Lys Phe Phe Val Pro Pro Ser Glu Gln
               500                 505                 510
   Ser Leu Arg Ala Val Ala
           515

<210>  9
<211>  124
<212>  PRT
<213>  Arachis hypogaea

<400>  9

Met Arg Arg Glu Arg Gly Arg Gln Gly Asp Ser Ser Ser Cys Glu Arg
1               5                   10                  15

Gln Val Asp Arg Val Asn Leu Lys Pro Cys Glu Gln His Ile Met Gln
            20                  25                  30

Arg Ile Met Gly Glu Gln Glu Gln Tyr Asp Ser Tyr Asp Ile Arg Ser
        35                  40                  45

Thr Arg Ser Ser Asp Gln Gln Gln Arg Cys Cys Asp Glu Leu Asn Glu
    50                  55                  60

Met Glu Asn Thr Gln Arg Cys Met Cys Glu Ala Leu Gln Gln Ile Met
65                  70                  75                  80

Glu Asn Gln Cys Asp Arg Leu Gln Asp Arg Gln Met Val Gln Gln Phe
                85                  90                  95

Lys Arg Glu Leu Met Asn Leu Pro Gln Gln Cys Asn Phe Arg Ala Pro
            100                 105                 110

Gln Arg Cys Asp Leu Asp Val Ser Gly Gly Arg Cys
        115                 120

## Description

The present invention relates to hypoallergenic protein variants derived from allergenic 2S albumin and cupin storage proteins of peanuts, tree nuts and seeds by introducing sequence and structural changes into one or more regions of the wildtype proteins.

Allergen-specific therapeutic approaches currently under investigation in mouse models include the use of mutated recombinant proteins, T- or B-cell based peptide immunotherapy or plasmid DNA-based immunotherapy. Current theory hypothesizes that allergens should be administrated in high doses to shift the allergen-specific T-cell cytokine production from a proallergic Th2 profile to an antiallergic Th1 type profile. The use of recombinant wild-type allergens poses the risk of adverse allergic reactions as a consequence of their reactivity with preformed IgE. Therefore, recombinant food allergens need to display a highly reduced capacity to react with such IgE bound to mast cells or basophils, ensuring a much lower risk of IgE-mediated side-effects.

In US 7,879,977 it is disclosed that the IgE binding sites of an allergen can be converted in vitro by site-directed mutagenesis to non-IgE binding sites by altering as little as a single amino acid residue within the epitope (preferably a hydrophobic one) to reduce or eliminate IgE binding. The linear IgE epitopes of the peanut allergens Ara h 1 (WO 99/45961), Ara h 2 (US 6,486,311) and Ara h 3 (US 7,485,708) were mapped using overlapping peptides and pooled serum IgE from peanut allergic patients. The average decrease in IgE binding to the modified allergens compared to the wild-type allergens was 35% for Ara h 1, 71% for Ara h 2 and 41% for Ara h 3.

However, similar results were obtained with reduced and alkylated Ara h 2, which in comparison to native Ara h 2, displayed reduced IgE binding in immunoblot analysis and a drastically reduced capacity to induce mediator release from humanized RBL cells. These results indicate the predominance of conformational IgE epitopes on Ara h 2 and only a minor involvement of sequential epitopes as predicted in US 7,879,977.

In WO 2005/060994 A1 it has been disclosed that food allergens, in particular seed storage proteins such as 2S albumins, may be modified by reduction and alkylation. It is postulated that this treatment results in both breakage of disulfide bonds and prevention of reformation of these disulfide bonds. The modification is stated to result in a reduction or even prevention of the production of specific IgE antibodies.

Similarly, WO 2010/000873 A1 describes an improved way of modifying allergens, wherein the peanut allergens Ara h 2 and Ara h 6 are reduced, alkylated and cross-linked by glutaraldehyde. This double modification of these allergens leads to a decreased IgE-binding when compared to the native allergens as demonstrated by IgE ELISA, IgE immunoblot and basophil histamine release. However, only allergens possessing cysteine residues that form disulfide bridges are suitable for this modification. Furthermore, treatment with chemically modified allergens can provide a clinical benefit but the methods for their preparation are poorly standardized and the characterization of the so-called allergoids is difficult. Additionally, reduction of disulfide bridges of highly structured proteins leads to their decreased solubility.

Food allergy is an IgE-mediated persisting immune disorder that is of major concern worldwide, since food allergies in a major way negatively affect the quality of life of patients with food allergy and their families. There is currently no routine immunotherapy available to treat often severe and sometimes fatal food allergy. The present standard of allergy care includes appropriate diagnosis, avoidance of the food, and education of the patient and family. But the aspired goal is sustained oral tolerance rather than desensitization. Most therapies are unable to achieve this as they are still in their relative infancy. Traditional subcutaneous allergen immunotherapy involves repeated injections of crude extracts over a long period of time and the high risk of severe systemic side-effects. Although attempts, such as oral immunotherapy, to desensitize patients with food allergy date back more than 100 years, there are no accepted therapies that accelerate the development of oral tolerance or provide effective protection from unintentional exposures. Consequently, there is a vital need for new therapeutic modalities. A number of therapeutic strategies are under investigation targeting the foods (peanut, tree nuts, and shellfish) that most frequently provoke severe IgE-mediated anaphylactic reactions. Therapeutic options should be safe, easily administered, and relatively inexpensive.

It is one of the objectives of the present invention to provide means and methods for genetically engineering hypoallergenic molecules for the use in allergen specific immunotherapy, for example for the immunotherapy of food allergy such as peanut allergy.

Therefore, the present invention relates to a method for producing/obtaining a hypoallergenic variant of an allergenic polypeptide having one or more loop regions comprising the step of modifying at least one loop region within the allergenic polypeptide by dividing said at least one loop region into at least two fragments, each of said fragments comprising at least 4 amino acid residues and combining said at least two fragments in different order than in said allergenic polypeptide.

Hypoallergenic molecules derived from an allergenic polypeptide show by definition a reduced IgE binding capacity compared to the polypeptide from which they are derived. Such hypoallergenic molecules can be obtained by various methods known in the art and are used in the prevention and/or the treatment of individuals suffering from an allergic condition in response to an allergenic polypeptide. In order to provoke a specific immune response against an allergenic polypeptide it is advantageous to retain in a hypoallergenic molecule not only the T-cell epitopes present in the allergenic polypeptide but also its three-dimensional structure. This is required to preserve the core of the molecule which is essential for its stability and solubility. It turned out that the three-dimensional structure of a wildtype allergenic polypeptide can be preserved in a hypoallergenic molecule if one or more loop regions within the allergenic polypeptide are divided into at least two fragments and rejoined in the same loop but in a different order than in said allergenic polypeptide. It is surprising that a molecule obtainable by such a method exhibits a reduced IgE binding capacity although the three-dimensional structure of the allergenic polypeptide is preserved and fragments present in the allergenic polypeptide are conserved.

The at least one loop region can also be divided in more than 2 fragments (preferably 3, 4, 5, 6, 7, 8, 9 or 10). The number of possible fragments to be rejoined in the hypoallergenic variant varies of course depending on the length of the loop region to be modified. For instance, if the loop region comprises more than 20 amino acid residues it would be possible to divide said loop region into 3, 4 or 5 fragments.

As used herein, a "loop region" is a portion of peptide sequence that extends either from or between an α-helical or a β-strand conformation or α-helical or β-strand conformations. As used herein, an α-helical or a "β-strand region" contains an extended α-helical or β-strand conformation, i.e. α-helices or β-strands comprising at least 4, preferably at least 5 amino acid residues. Loop regions are typically free of extended α-helical or β-strand conformations but may include shortened α-helical or β-strand conformations, i.e. α-helices or β-strands comprising less than 4 amino acids. Apart from N- and C-terminal loop regions, the loop regions connect α-helices or β-strands running in opposite directions. The loop regions of a protein with unknown three-dimensional structure can be determined by amino acid sequence alignment with sequences from homologous proteins with known experimentally determined structures. In addition, the loop regions of a protein with unknown three-dimensional structure can be determined by homology modelling using homologous proteins with known experimentally determined structures as templates.(Refs.: Eswar N, et al. Methods Mol Biol. 426(2008):145-159; Arnold K. et al. Bioinformatics, 22(2006):,195-201; Qu X et al. Curr Protein Pept Sci. 10 (2009) :270-285)

"Dividing", as used herein, means to divide or split an amino acid stretch comprising a number of amino acid residues into smaller fragments. According to the present invention said fragments comprise at least 4 consecutive amino acid residues present in at least one loop region of an allergenic polypeptide or protein.

"Combining", as used herein, means that an amino acid stretch comprising a number of amino acid residues is (re-)introduced into the loop region of a hypoallergenic variant. However the order or sequence of the fragments in the hypoallergenic variant is different from that in the allergenic polypeptide. For instance, a loop comprising fragments A-B-C-D in the allergenic polypeptide could be combined in the hypoallergenic variant to have the following orders/sequences: B-A-D-C, D-B-C-A, C-B-A-D, A-C-B-D, A-D-B-C, A-D-C-B etc. Of course it is also possible to combine fragments of various loop regions within the protein. In such a case an entire loop region or one or more fragments thereof is/are exchanged with another loop region within the same protein. The exchange of the loops or fragments thereof should not influence significantly the stability and solubility of the allergen variant obtained from this exchange.

The term "hypoallergenic" as used herein refers to the ability of the variants of the present invention to exhibit reduced or no allergic reactions when administered to an individual (reduced or no IgE reactivity compared to the wild-type allergenic polypeptide)and to induce the production of IgG antibodies specifically binding to said allergen. The reduced or missing ability of "hypoallergenic" variants of an allergenic polypeptide to induce an allergic reaction in an individual is obtained by removing or destroying the IgE binding epitopes from said allergenic polypeptide, but conserving the T-cell epitopes present on said allergenic polypeptide as well as its three-dimensional structure.

The hypoallergenic variants of the present invention can be obtained by using genetic engineering of nucleic acid molecules encoding the allergenic polypeptide. Respective methods are known to the person skilled in the art.

According to a preferred embodiment of the present invention the at least one loop region to be modified comprises at least 10 amino acid residues.

According to another preferred embodiment of the present invention the at least one loop region is divided into at least two fragments, each of said fragments comprising a maximum of 20, preferably 15, more preferably 10, amino acid residues.

The at least one loop region is preferably divided into at least two fragments, each of said fragments comprising 4 to 20, preferably 5 to 15, more preferably 5 to 10, amino acid residues.

According to a preferred embodiment of the present invention the allergenic polypeptide comprises disulfide bonds.

Disulfide bonds stabilise the three-dimensional structure of polypeptides and proteins. Such bonds are formed by cysteine residues present in an allergenic polypeptide as defined herein. Of course the method of the present invention can also be used with proteins having no disulfide bonds.

According to another preferred embodiment of the present invention the hypoallergenic variant exhibits an at least 10%, preferably an at least 20%, more preferably an at least 40%, even more preferably an at least 50%, most preferably an at least 70%, reduced IgE binding capacity compared to the allergenic polypeptide from which said hypoallergenic variant has been derived.

"IgE binding capacity", as used herein, is the capacity of a molecule to bind to IgE which is able to bind to a specific allergenic polypeptide. The IgE binding capacity of polypeptides and proteins can be determined by, for example, an enzyme linked immunosorbent assay (ELISA) using, for example, sera obtained from an individual (i.e. an allergic individual) who has been previously exposed to the wildtype allergenic polypeptide. Briefly, a polypeptide or protein to be tested is coated onto wells of a microtiter plate. After washing and blocking the wells, antibody solution consisting of the serum or plasma of an allergic individual who has been exposed to the molecule being tested or the protein from which it was derived is incubated in the wells. The plasma is generally depleted of IgG before incubation. However, the depletion is not necessary if highly specific anti IgE-antibodies are used. A labelled secondary antibody is added to the wells and incubated. The amount of IgE binding is then quantified and compared to the amount of IgE bound by a purified wildtype allergenic polypeptide or protein. Alternatively, the binding capacity of a molecule can be determined by Western blot analysis. For example, a peptide to be tested is run on a polyacrylamide gel using SDS-PAGE. The peptide is then transferred to nitrocellulose and subsequently incubated with sera from allergic subjects. After incubation with the labelled secondary antibody, the amount of IgE bound is then determined and quantified.

Another assay which can be used to determine the IgE binding capacity of a polypeptide or protein is a competition ELISA assay. Briefly, an IgE antibody pool is generated by combining plasma or serum from allergic individuals that have been shown by direct ELISA to have IgE reactive with wildtype allergenic polypeptide. This pool is used in ELISA competition assays to compare IgE binding to wild-type allergenic polypeptide to the hypoallergenic variant. IgE binding to the allergenic polypeptide and the molecule being tested is determined and quantified.

Furthermore, the hypoallergenic variants of the present invention preferably do not result in the release of mediators (e.g. histamine) from mast cells or basophils. To determine whether a polypeptide which binds IgE results in the release of mediators, a histamine release assay can be performed using standard reagents and protocols. Briefly, a buffered solution of a polypeptide to be tested is combined with an equal volume of whole heparinized blood from an allergic subject. After mixing and incubation the cells are pelleted and the supernatants are processed and analyzed using, e.g., a radioimmunoassay to determine the amount of histamine released.

According to a preferred embodiment of the present invention allergenic polypeptide is an allergenic prolamin, preferably a member of the 2S albumin group and also including allergenic bifunctional amylase/protease inhibitors and allergenic non-specific lipid transfer proteins, or an allergenic cupin, preferably selected from the groups of allergenic 7S globulins (vicilins) or 11S globulins (legumins).

The prolamin superfamily derives its name from the alcohol-soluble proline- and glutamine-rich storage proteins of cereals. Allergenic members of this superfamily are characterized by the presence of a bundle of α-helices that contain a conserved pattern of eight or ten cysteine residues that form four or five intramolecular disulfide bonds. Apart from the conserved cysteine pattern, there exist few sequence similarities between members of the different families. Members of the prolamin superfamily include the cereal prolamin seed storage proteins and several families of disulfide-rich small proteins. The prolamin seed storage proteins (gliadins and glutenins) contain a repetitive coiled-coil domain rich in proline and glutamine residues and a globular disulfide-rich domain. Families of low molecular weight sulfur-rich proteins are the grain softness proteins, indolines, non-specific lipid transfer proteins, soybean hydrophobic protein, bifunctional α-amylase/protease inhibitors, and 2S albumin seed storage proteins.

The allergenic 2S albumin is preferably derived from a plant selected from the group consisting of *Arachis hypogaea,* Glycine max, *Lupinus albus, Lupinus angustifolius, Corylus avellana, Juglans regia, Juglans nigra, Carya illinoinensis, Anacardium occidental, Pistacia vera, Bertolletia excels, Fagopyrum esculentum, Fagopyrum tataricum, Sesamum indicum, Sinapis alba, Brassica juncea, Brassica napus, Brassica* rapa and *Ricinus communis.*

The allergenic 2S albumin is preferably selected from the group consisting of Ara h 2, Ara h 6, Ara h 7, Gly m 2S albumin, Lup a delta_Conglutin, Lup an delta_Conglutin, Cor a 14, Jug r 1, Jug n 1, Car i 1, Ana o 3, Pis v 1, Ber e 1, Fag e 2, Fag t 2, Ses i 1, Ses i 2, Sin a 1, Bra j 1, Bra n 1, Bra r 1 and Ric c 1.

The allergenic 7S globulin is preferably derived from a plant selected from the group consisting of *Arachis hypogaea, Glycine max, Pisum sativum, Lens culinaris, Lupinus albus, Lupinus angustifolius, Corylus avellana, Juglans regia, Juglans nigra, Anacardium occidentale, Pistacia vera, Fagopyrum esculentum* and *Sesamum indicum.*

The allergenic 7S globulin is preferably selected from the group consisting of Ara h 1, Gly m 5, Pis s 1, Pis s 2, Len c 1, Lup a 1, Lup an 1, Cor a 11, Jug r 2, Jug n 2, Ana o 1, Pis v 3, Fag e 19kD and Ses i 3.

The allergenic 11S globulin is preferably derived from a plant selected from the group consisting of *Arachis hypogaea, Glycine max, Lupinus albus, Lupinus angustifolius, Corylus avellana, Juglans regia, Carya illinoinensis, Anacardium occidentale, Pistacia vera, Bertolletia excels, Prunus dulcis, Fagopyrum esculentum, Fagopyrum tataricum, Sesamum indicum* and *Sinapis alba.*

The allergenic 11S globulin is preferably selected from the group consisting of Ara h 3, Ara h 4, Gly m 6, Lup a alpha_Conglutin, Lup an alpha_Conglutin, Cor a 9, Jug r 4, Car i 4, Ana o 2, Pis v 2, Pis v 5, Ber e 2, Pru du 6, Fag e 1, Fag t 1, Ses i 6, Ses i 7 and Sin a 2.

The allergenic bifunctional amylase/protease inhibitor is preferably derived from a plant selected from the group consisting of *Triticum aestivum, Hordeum vulgare* and *Oryza sativa.*

The allergenic bifunctional amylase/protease inhibitor is preferably selected from the group consisting of Tri a 15, Tri a 28, Tri a 29, Tri a 30, Hor v 15, BDAI-I, BTI-CMe, RAG1 and RAG2.

The allergenic non-specific lipid transfer protein is preferably derived from a plant selected from the group consisting of *Parietaria judaica, Platanus orientalis, Ambrosia artemisifolia, Artemisia vulgaris, Helianthus annuus, Lactuca sativa, Hevea brasiliensis, Prunus persica, Prunus armeniaca, Prunus avium, Prunus domestica, Prunus dulcis, Malus domestica, Pyrus communis, Fragaria ananassa, Rubus idaeus, Morus nigra, Vitis vinifera, Citrus sinensis, Arachis hypogaea, Lens culinaris, Phaseolus vulgaris, Corylus avellana, Juglans regia, Triticum aestivum, Triticum durum, Herdeum vulgare, Oryza sativa, Zea mays, Apium graveolens, Daucus carota, Brassica oleracea, Brassica rapa, Sinapis alba* and *Solanum lycopersicum.*

The allergenic non-specific lipid transfer protein is preferably selected from the group consisting of Par j 1, Par j 2, Pla or 3, Amb a 6, Art v 3, Hel a 3, Lac s 1, Hev b 12, Pru p 3, Pru ar 3, Pru av 3, Pru d 3, Pru du 3, Mal d 3, Pyr c 3, Fra a 3, Rub i 3, Mor n 3, Vit v 1, Cit s 3, Ara h 9, Len c 3, Pha v 3, Cor a 8, Jug r 3, Tri a 14, Tri td 14, Hor v 14, Ory s 14, Zea m 14, Api g 2, Dau c 3, Bra o 3, Bra r 3, Sin a 3 and Lyc e 3.

Particularly preferred allergenic polypeptides are 2S albumins. All 2S albumins share a common fold: 4-5 α-helices form a bundle stabilized by 4 to 5 disulfide bridges. The α-helices are connected by short turns (around 5 amino acids) or long loops (>10 amino acids). The IgE binding ability of these loops, which form the major part of the antibody-accessible surface of these proteins, can be reduced partially (at least 10%, preferably at least 50%) or substantially entirely using the method of the present invention. In contrast, the α-helices and disulfide bonds were left unchanged in order to obtain soluble, stably folded proteins. Table 1 shows a list of allergenic 2S albumins with known sequence, for which this mutation strategy is applicable.

The secondary structure content of the allergen of interest can be derived from its experimentally determined structure or obtained by homology modeling using known structures of homologous proteins with, e.g., >30% sequence identity as templates. Helices, including one extra residue each at their N-and C-termini, short turns (less than 10 amino acid residues) and cysteine residues remain unchanged. Long loops (with at least 10 amino acid residues) between unchanged parts are mutated as follows: in each loop, the sequence is split into short stretches of 4 to 20, preferably 5 to 10 amino acid residues in length. In loops that comprise 2 segments, the order of these segments is swapped, i.e. the N-terminus of the second segment is connected to the C-terminus of the preceding helix, the N-terminus of the first segment is connected to the C-terminus of the second segment, and the C-terminus of the first segment is connected to the N-terminus of the succeeding helix. In loops that comprise three segments, the first and third segments are swapped while leaving the central segment in place. In loops comprising more than three segments, the region is preferably split into parts containing 2 to 3 segments, before applying the procedure as outlined above to each part.

Another preferred group of allergenic polypeptides are allergenic 7S and 11S globulins.

7S and 11S globulins share the typical fold of members of the cupin superfamily. The structural core of the molecule consists of two beta barrels composed of 10 to 12 anti-parallel beta-strands. The beta-strands within each barrel and the two barrels are connected either by short (less than 10 amino acid residues) turns or long sequences composed of α-helical and random coil segments. The following mutation strategy is devised with the aim of reducing the IgE-binding ability of these loop regions, which comprise the major part of the antibody-accessible surface of the protein. In contrast, the cupin barrels are preferably left unchanged in order to obtain soluble, stably folded proteins. Table 2 and Table 3 show a list of allergenic 7S and 11S globulins with known sequence, for which this mutation strategy is applicable.

The secondary structure content of the allergen of interest is derived from its experimentally determined structure or obtained by homology modeling using known structures of homologous proteins with, e.g., more than 30% sequence identity as templates. Beta-strands, including one extra residue each at their N- and C-termini and short turns (less than 10 amino acid residues) remain unchanged. Long loops (>= 10 amino acids) between unchanged parts can be mutated as follows: in each loop, the sequence is split into short stretches of 5 to 10 residues in length. In loops that comprised 2 segments, the order of these segments is swapped, i.e. the N-terminus of the second segment is connected to the C-terminus of the preceding helix, the N-terminus of the first segment is connected to the C-terminus of the second segment, and the C-terminus of the first segment is connected to the N-terminus of the succeeding helix. In loops that comprise, e.g., three segments, the first and third segments are swapped while leaving the central segment in place. In loops comprising more than three segments, the region can be split into parts containing 2 to 3 segments, before applying the procedure outlined above to each part.

Since all families of allergens from the prolamin superfamily share the same architecture of a disulfide-stabilized bundle of α-helices connected by long loops, the method of the present invention can be applied to the family of cereal bifunctional inhibitors of amylases and proteases, such as Tri a 15 from wheat and Hor v 15 from barley (Table 4) and to the family of non-specific lipid transfer proteins, such as Pru p 3 from peach and Cor a 8 from hazelnut (Table 5).

Particularly preferred allergens which are modified according to a method of the present invention to obtain hypoallergenic variants thereof are listed in tables 1 to 5.

**Table 1: Allergenic 2S albumins with known sequences**

| **Source** | | **Allergen name** | **UniProt accession numbers** |
|---|---|---|---|
| Fabaceae (legumes) | | | |
| | Peanut (*Arachis hypogaea*) | **Ara h 2** | Q6PSU2 |
| | | **Ara h 6** | Q647G9 |
| | | **Ara h 7** | Q9SQH1, B4XID4 |
| | Soybean (*Glycine max*) | Gly m 2S albumin | P19594 |
| | White lupin (*Lupinus albus*) | Lup a delta_Conglutin | Q333K7 |
| | Blue lupin (*Lupinus angustifolius*) | Lup an delta_Conglutin | P09930, P09931, Q99235 |
| Betulaceae (birch family) | | | |
| Hazelnut (*Corylus avellana*) | | **Cor a 14** | DOPWG2 |
| Juglandaceae (walnut family) | | | |
| | Common walnut (*Juglans regia*) | **Jug r 1** | P93198 |
| | Black walnut (*Juglans nigra*) | **Jug n 1** | Q7Y1C2 |
| | Pecan nut (*Carya illinoinensis*) | **Car i 1** | Q84XA9 |
| Anacardiaceae (sumac family) | | | |
| | Cashew nut (*Anacardium occidentale*) | **Ana o 3** | Q8H2B8 |
| | Pistachio (*Pistacia vera*) | **Pis v 1** | B7P072 |
| Lecythidaceae | | | |
| | Brazil nut (Bertholletia *excelsa*) | **Ber e 1** | P04403 |
| Polygonaceae (knotweed family) | | | |
| | Common buckwheat (*Fagopyrum esculentum*) | **Fag e 2** | Q2PS07 |
| | Tartary buckwheat (*Fagopyrum tataricum*) | **Fag t 2** | E9NX73 |
| Pedaliaceae (sesame family) | | | |
| | Sesame (*Sesamum indicum*) | **Ses i 1** | Q9AUD1 |
| | | **Ses i 2** | Q9XHP1 |
| Brassicaceae (cabbage family) | | | |
| | White mustard (*Sinapis alba*) | **Sin a 1** | P15322 |
| | Indian mustard *(Brassica juncea*) | **Bra j 1** | P80207 |
| | Rapeseed (*Brassica napus*) | **Bra n 1** | P80208 |
| | Turnip (*Brassica rapa*) | **Bra r 1** | Q42473 |
| Euphorbiaceae (spurge family) | | | |
| | Castor bean (*Ricinus communis*) | **Ric c 1** | P01089 |

| | | | |
|---|---|---|---|
| Bold: allergens officially approved by the IUIS Allergen Nomenclature Subcommittee (www.allergen.org) Plain: Additional potential allergens recorded in Allergome (www.allergome.org) | | | |

**Table 2: Allergenic 7S globulins with known sequences**

| **Source** | | **Allergen name** | **UniProt accession numbers** |
|---|---|---|---|
| Fabaceae (legumes) | | | |
| | Peanut (*Arachis hypogaea*) | **Ara h 1** | P43238 |
| | Soybean (*Glycine max*) | **Gly m 5** | 022120, Q9FZP9, P25974 |
| | Pea (*Pisum sati vum*) | **Pis s 1** | Q702P1, Q702P0 |
| | | **Pis s 2** | P13915 |
| | Lentil (*Lens culinaris*) | **Len c 1** | Q84UI1, Q84UI0 |
| | White lupin (*Lupinus albus*) | Lup a 1 | Q53HY0, Q6EBC1 |
| | Blue lupin (*Lupinus angustifolius*) | **Lup an 1** | B8Q5G0 |
| Betulaceae (birch family) | | | |
| | Hazelnut (*Corylus avellana*) | **Cor a 11** | Q8S4P9 |
| Juglandaceae (walnut family) | | | |
| | Common walnut (*Juglans regia*) | **Jug r 2** | Q9SEW4 |
| | Black walnut (*Juglans nigra*) | **Jug n 2** | Q7Y1C1 |
| Anacardiaceae (sumac family) | | | |
| | Cashew nut (*Anacardium occidentale*) | **Ana o 1** | Q8L5L5, Q8L5L6 |
| | Pistachio (*Pistacia vera*) | **Pis v 3** | B4X640 |
| Polygonaceae (knotweed family) | | | |
| | Common buckwheat (*Fagopyrum esculentum*) | Fag e 19kD | A5HIX6 |
| Pedaliaceae (sesame family) | | | |
| | Sesame (*Sesamum indicum*) | **Ses i 3** | Q9AUD0 |

| | | | |
|---|---|---|---|
| Bold: allergens officially approved by the IUIS Allergen Nomenclature Subcommittee (www.allergen.org) Plain: Additional potential allergens recorded in Allergome (www.allergome.org) | | | |

**Table 3: Allergenic 11S globulins with known sequences**

| | **Source** | **Allergen name** | **UniProt accession numbers** |
|---|---|---|---|
| | Fabaceae (legumes) | | |

| **Source** | | **Allergen name** | **UniProt accession numbers** |
|---|---|---|---|
| | Peanut (*Arachis hypogaea*) | **Ara h 3/4** | 082580, Q9SQH7 |
| | Soybean (*Glycine max*) | **Gly m 6** | P04776, P04405, P11828, Q9SB11, Q7GC77 |
| | White lupin (*Lupinus albus)* | Lup a alpha_Conglutin | Q53I54, Q53I55 |
| | Blue lupin (*Lupinus angustifolius*) | Lup an alpha_Conglutin | Q96475 |
| Betulaceae (birch family) | | | |
| | Hazelnut (*Corylus avellana*) | **Cor a 9** | Q8W1C2 |
| Juglandaceae (walnut family) | | | |
| | Common walnut (*Juglans regia*) | **Jug r 4** | Q2TPW5 |
| | Pecan nut (*Carya illinoinensis*) | **Car i 4** | B5KVH4 |
| Anacardiaceae (sumac family) | | | |
| | Cashew nut (*Anacardium occidentale*) | **Ana o 2** | Q8GZP6 |
| | Pistachio (*Pistacia vera*) | **Pis v 2** | B7P073, B7P074 |
| | | **Pis v 5** | B7SLJ1 |
| Lecythidaceae | | | |
| | Brazil nut (Bertolletia *excelsa*) | **Ber e 2** | Q84ND2 |
| Rosaceae (rose family) | | | |
| | Almond (*Prinis dulcis*) | **Pru du 6** | E3SH28, E3SH29 |
| Polygonaceae (knotweed family) | | | |
| | Common buckwheat (*Fagopyrum esculentum*) | Fag e 1 | 023878, 023880, P83004, Q9XFM4 |
| | Tartary buckwheat (*Fagopyrum tataricum*) | Fag t 1 | A9NJG2, Q8LGR7 |
| Pedaliaceae (sesame family) | | | |
| | Sesame (*Sesamum indicum*) | **Ses i 6** | Q9XHP0 |
| | | **Ses i 7** | Q9AUD2 |
| Brassicaceae (cabbage family) | | | |

| | **Source** | **Allergen name** | **UniProt accession numbers** |
|---|---|---|---|
| | White mustard (*Sinapis alba*) | **Sin a 2** | Q2TLW0 |

| | | | |
|---|---|---|---|
| Bold: allergens officially approved by the IUIS Allergen Nomenclature Subcommittee (www.allergen.org) Plain: Additional potential allergens recorded in Allergome (www.allergome.org) | | | |

**Table 4: Allergenic bifunctional amylase/protease inhibitors with known sequence**

| **Source** | | **Allergen name** | **UniProt accession numbers** |
|---|---|---|---|
| Poaceae (grasses) | | | |
| | Wheat (*Triticum aestivum*) | **Tri a 15** | D2TGC3 |
| | | **Tri a 28** | Q4W0V7 |
| | | **Tri a 29** | C7C4X0, D2TGC2 |
| | | **Tri a 30** | P17314 |
| | Barley (*Hordeum vulgare*) | **Hor v 15** | P16968 |
| | | BDAI-I | P13691, Q546U1 |
| | | BTI-CMe | P01086 |
| | Rice (*Oryza sativa*) | RAG1/RAG2 | Q01881, Q01882, Q01883 |

| | | | |
|---|---|---|---|
| Bold: allergens officially approved by the IUIS Allergen Nomenclature Subcommittee (www.allergen.org) Plain: Additional potential allergens recorded in Allergome (www.allergome.org) | | | |

**Table 5: Allergenic non-specific lipid transfer proteins with known sequences**

| **Source** | | **Allergen name** | **UniProt accession numbers** |
|---|---|---|---|
| Urticaceae (nettle family) | | | |
| | Pellitory of the wall (*Parietaria judaica*) | **Par j 1** | P43217, 004404, Q1JTN5, Q40905 |
| Platanaceae (plane trees) | | **Par j 2** | P55958, 004403 |
| | Oriental plane tree (*Platanus orientalis*) | **Pla or 3** | A9YUH6 |
| Asteraceae (sunflower family) | | | |
| | Short ragweed (*Ambrosia artemisifolia*) | **Amb a 6** | 004004 |
| | Mugwort (*Artemisia vulgaris*) | **Art** v **3** | P0C088, C4MGG9, C4MGH0, C4MGH1 |
| | Sunflower seed (*Helianthus annuus*) | **Hel a 3** | P82007 |
| | Garden lettuce (*Lactuca sativa*) | **Lac s 1** | A1E2H4, A1E2H5 |
| Euphorbiaceae (spurge family) | | | |
| | Para rubber tree (Hevea *brasiliensis*) | **Hev b 12** | Q8RYA8 |
| Rosaceae (rose family) | | | |
| | Peach (*Prunus persica*) | **Pru p** 3 | P81402, Q9LED1 |
| | Apricot (*Prunus armeniaca*) | **Pru ar 3** | P81651 |
| | Cherry (*Prunus avium*) | **Pru av 3** | Q9M5X8 |
| | Plum (*Prunus domestica*) | **Pru d 3** | P82534 |
| | Prunus dulcis (almond) | **Pru du 3** | C0L0I5 |
| | Apple (*Malus domestica*) | **Mal d 3** | Q9M5X7 |
| | Pear (*Pyrus communis*) | **Pyr c 3** | Q9M5X6 |
| | Strawberry (*Fragaria ananassa*) | **Fra a 3** | Q8VX12, Q4PLT9, Q4PLU0, Q4PLT6, Q4PLT8, Q4PLT7, Q4PLT5 |
| | Raspberry (*Rubus idaeus*) | **Rub i 3** | Q0Z8V0 |
| Moraceae (mulberry family) | | | |
| | Black mulberry (*Morus nigra*) | **Mor n 3** | P85894 |
| Vitaceae (grape family) | | | |
| | Grape (*Vitis vinifera*) | **Vit v 1** | P80274 |
| Rutaceae (rue family) | | | |
| | Sweet orange (*Citrus sinensis*) | **Cit s 3** | P84161, Q6EV47 |
| Fabaceae (legumes) | | | |
| | Peanut (*Arachis hypogaea*) | **Ara h 9** | B6CEX8, B6CG41 |
| | Lentil (*Lens culinaris*) | **Len c 3** | A0AT29 |
| | French bean (*Phaseolus vulgaris*) | **Pha v 3** | D3W146, D3W147 |
| Betulaceae (birch family) | | | |
| | Hazelnut (*Corylus avellana*) | **Cor a 8** | Q9ATH2 |
| Juglandaceae (walnut family) | | | |
| | Common walnut (*Juglans regia*) | **Jug r 3** | C5H617 |
| Poaceae (grasses) | | | |
| | Wheat (*Triticum aestivum*) | **Tri a 14** | D2T2K2 |
| | Durum wheat (*Triticum durum)* | Tri td 14 | Q5NE26, Q9FEK9 |
| | Barley (*Herdeum vulgare*) | Hor v 14 | Q8RYA8 |
| | Rice (*Oryza sativa*) | Ory s 14 | QOIQK9 |
| | Maize (*Zea mays*) | **Zea m 14** | P19656 |
| Apiaceae (parsley family) | | | |
| | Celeriac (*Apium graveolens*) | **Api g 2** | E6Y8S8 |
| | Carrot (*Daucus carota*) | Dau c 3 | P27631 |
| Brassicaceae (cabbage family) | | | |
| | Cabbage (*Brassica oleracea*) | **Bra o 3** | Q39382 |
| | Turnip (*Brassica* rapa) | Bra r 3 | 064431 |
| | White mustard (*Sinapis alba*) | **Sin a 3** | E6Y2L9 |
| Solanaceae (nightshade family) | | | |
| | Tomato (*Solanum lycopersicum*) | **Lyc e 3** | P93224 |

| | | | |
|---|---|---|---|
| Bold: allergens officially approved by the IUIS Allergen Nomenclature Subcommittee (www.allergen.org) Plain: Additional potential allergens recorded in Allergome (www.allergome.org) | | | |

The allergenic polypeptide Ara h 1, an allergen of the peanut, has preferably the following amino acid sequence SEQ ID No. 6:

RQPRREEGGRWGPAGPREREREEDWRQPREDWRRPSHQQPRKIRPEGREGEQEWGTPGSHVREE

TSRNNPFYFPSRRFSTRYGNQNGRIRVLQRFDQRSRQFQNLQNHRIVQIEAKPNTLVLPKHADA
DNILVIQQGQATVTVANGNNRKSFNLDEGHALRIPSGFISYILNRHDNQNLRVAKISMPVNTPG
QFEDFFPASSRDQSSYLQGFSRNTLEAAFNAEFNEIRRVLLEENAGGEQEERGQRRWSTRSSEN
NEGVIVKVSKEHVEELTKHAKSVSKKGSEEEGDITNPINLREGEPDLSNNFGKLFEVKPDKKNP
QLQDLDMMLTCVEIKEGALMLPHFNSKAMVIVVVNKGTGNLELVAVRKEQQQRGRREEEEDEDE
EEEGSNREVRRYTARLKEGDVFIMPAAHPVAINASSELHLLGFGINAENNHRIFLAGDKDNVID
QIEKQAKDLAFPGSGEQVEKLIKNQKESHFVSARPQSQSQSPSSPEKESPEKEDQEEENQGGKG
PLLSILKAFN

According to a preferred embodiment of the present invention the loop regions of Ara h 1 to be modified and thus the loop regions comprising the fragments of Ara h 1 to be re-ordered are selected from the group consisting of amino acid residues 1 to 40, 48 to 56, 93 to 107, 209 to 233, 241 to 249, 271 to 285, 369 to 376, 383 to 390, 447 to 454, 461 to 468, 484 to 508 and 514 to 522 of SEQ ID No. 6 and combinations thereof. It is particularly preferred that at least 2, at least 3, at least 5, at least 10 or even all of said regions are modified.

The mature allergenic polypeptide Ara h 2, an allergen of the peanut, has preferably the following amino acid sequence SEQ ID No. 7:

RQQWELQGDRRCQSQLERANLRPCEQHLMQKIQRDEDSYGRDPYSPSQDPYSPSQDPDRRDPYS
PSPYDRRGAGSSQHQERCCNELNEFENNQRCMCEALQQIMENQSDRLQGRQQEQQFKRELRNLP
QQCGLRAPQRCDLEVESGGRDRY

According to a preferred embodiment of the present invention the loop regions of Ara h 2 to be modified and thus the loop regions comprising the fragments of Ara h 2 to be re-ordered are selected from the group consisting of amino acid residues 1 to 10, 34 to 73, 132 to 137 and 140 to 145 of SEQ ID No. 7 and combinations thereof. It is particularly preferred that at least 2, at least 3, at least 5, at least 10 or even all of said regions are modified.

The mature allergenic polypeptide Ara h 3, an allergen of the peanut, has preferably the following amino acid sequence SEQ ID No. 8:

ISFQRLNAQRPFRQQPEENACQDNRIESEGGYIETWFECAGNPNNQEVALSRLVLRRNALRRPF
YSNAPQEIFIQQGRGYFGLIFPGCPSTYEEPAQQGRRHQSQRPPRRFQGQDQSQQQQDSHQKVH
RFDEGDLIAVPTGVAFWMYNDHDTDVVAVSLTDTNNNDNQLDQFPRRFNLAGNHEQEFLRYQQQ
SRRRSLPYSPYSPQTQPKQEDREFSPRGQHGRRERAGQEQENEGGNIFSGFTPEFLAQAFQVDD
RQILQNLRGENESDEQGAIVTVRGGLRILSPDRKRRQQYERPDEEEEYDEDEYEYDEEERQQDR
RRGRGSRGSGNGIEETICTASFKKNIGRNRSPDIYNPQAGSLKTANELQLNLLILRWLGLSAEY
GNLYRNALFVPHYNTNAHSIIYALRGRAHVQVVDSNGDRVFDEELQEGHVLVVPQNFAVAGKSQ
SENFEYVAFKTDSRPSIANLAGENSFIDNLPEEVVANSYGLPREQARQLKNNNPFKFFVPPSEQ
SLRAVA

The mature allergenic polypeptide Ara h 6, an allergen of the peanut, has preferably the following amino acid sequence SEQ ID No. 9:

MRRERGRQGDSSSCERQVDRVNLKPCEQHIMQRIMGEQEQYDSYDIRSTRSSDQQQRCCDELNE
MENTQRCMCEALQQIMENQCDRLQDRQMVQQFKRELMNLPQQCNFRAPQRCDLDVSGGRC

According to a preferred embodiment of the present invention the loop regions of Ara h 6 to be modified and thus the loop regions comprising the fragments of Ara h 6 to be re-ordered are selected from the group consisting of amino acid residues 2 to 13, 36 to 49, 116 to 121 and 108 to 113 of SEQ ID No. 9 and combinations thereof. It is particularly preferred that at least 2, at least 3, at least 5, at least 10 or even all of said regions are modified.

A preferred hypoallergenic variant of the present invention being derived from Ara h 1 has preferably the following amino acid sequence (SEQ ID No. 1; the exchanged fragments within the loop regions are marked in bold and underlined or in italic and underlined):

GRWGPAGPRQPRREEGWRQPREDWREREREEDEGEQEWGTRKIRPEGRRRPSHQQPPGSHVREE
TSRNNPFYFPSRRFSTRYGNQNGRIRVLQQFQNLQNRFDQRSRHRIVQIEAKPNTLVLPKHADA
DNILVIQQGQATVTVANGNNRKSFNLDEGHALRIPSGFISYILNRHDNQNLRVAKISMPVNTPG
QFEDFFPASSRDQSSYLEAAFNAEFQGFSRNTLEERGQRRWEENAGGEQNEIRRVLLSTRSSEN
NEGVIVKVSKEHVEEVSKKGSELTKHAKSEEGDITNPINLREGEPDLSNNFGKLFEVKPDKKNP
QLQDLDMMLTCVEIKEGALMLPHFNSKAMVIVVVNKGTGNLELVAVRKEEEEEGSNEEEEDEDQ
QQRGRRREVRRYTARLKEGDVFIMPAAHPVAINASSELHLLGFGINAENNHRIFLAGDKDNVIS

GEQVEKKDLAFPGDQIEKQALIKNQKESHFVSARSSPEKESPPQSQSQSPLSILKAFNNQGGKG
PLEKEDQEEE

A preferred hypoallergenic variant of the present invention being derived from Ara h 2 has preferably the following amino acid sequence (SEQ ID No. 2; the exchanged fragments within the loop regions are marked in bold and underlined or in italic and underlined):

LQGDRRQQWERCQSQLERANLRPCEQHLMQKIQRSPSQDPYSPSDEDSYGRDPYPSPYDRRGAQ
DPDRRDPYSGSSQHQERCCNELNEFENNQRCMCEALQQIMENQSDRLQGRQQEQQFKRELRNLP
QQCGLEVESRCDLRAPQGGRDRY

An alternative preferred hypoallergenic variant of the present invention being derived from Ara h 2 has preferably the following amino acid sequence (SEQ ID No. 3; the exchanged fragments within the loop regions are marked in bold and underlined or in italic and underlined):

LQGDRRQQWERCQSQLERANLRPCEQHLMQKIQRRDPYSPDEDSYGSPSQDSQDPYPDRRDPYD
RRGAYSPSPGSSQHQERCCNELNEFENNQRCMCEALQQIMENQGRQLQSDRQEQQFKRELRNLP
QQCGLEVESRCDLRAPQGGRDRY

A preferred hypoallergenic variant of the present invention being derived from Ara h 3 has preferably the following amino acid sequence (SEQ ID No. 4; the exchanged fragments within the loop regions are marked in italic and underlined or in bold and underlined):

ISFQRLNAQRPFRQQPEENACQDNRIESEGGYIETWFECAGNPNNQEVALSRLVLRRNALRRPF
YSNAPQEIFIQQGRGYFGLIFPGCPSRRHQSQRPPTYEEPAQQGQQQQDSHQKRRFQGQDQSVH
RFDEGDLIAVPTGVAFWMYNDHDTDVVAVSLTDLDQFPRTNNNDNQRFNLLRYQQQSRAGNHEQ
EFYSPQTQPKRRSLPYSPRGQHGRREQEDREFSPEGGNIFSGRAGQEQENAFQVDDRQFTPEFL
AQNESDEQGILQNLRGEAIVTVPDRKRRQQRGGLRILSEYDEDEYEYERPDEEEYDEEERQQRG
SGNGIEDRRRGRGSETICTASFKKNIGRNRSPDIYNPQAGSLKTANILRWLGELQLNLLLSAEY
GNLYRNALFVPHYNTNAHSIIYALRGRAHVQVVDSNGDRVFDEELQEGHVLVVPQNFAVAGKSQ
SENFEYVAFKTDSRPSIANLDNLPEEVAGENSFIVANSYGLLKNNNPFKPREQARQFFVPPSEQ
SLRAVA

A preferred hypoallergenic variant of the present invention being derived from Ara h 6 has preferably the following amino acid sequence (SEQ ID No. 5; the exchanged fragments within the loop regions are marked in bold and underlined or in italic and underlined):

MRQGDSSRRERGSCERQVDRVNLKPCEQHIMQRIMGSYDIRSTEQEQYDRSSDQQQRCCDELNE
MENTQRCMCEALQQIMENQCDRLQDRQMVQQFKRELMNLPQQCNLDVSGRCDFRAPQGRC

Another aspect of the present invention relates to a hypoallergenic variant of an allergenic polypeptide having one or more loop regions obtainable by a method according to the present invention.

Yet another aspect of the present invention relates to a hypoallergenic variant of an allergenic polypeptide having one or more loop regions obtainable by modifying at least one loop region within the allergenic polypeptide by dividing said at least one loop region into at least two fragments, each of said fragments comprising at least 4 amino acid residues and combining said at least two fragments in another order than in said allergenic polypeptide.

Another aspect of the present invention relates to a hypoallergenic variant of an allergenic polypeptide having one or more loop regions, wherein at least two fragments of at least 4 amino acid residues of at least one loop region of the allergenic polypeptide are ordered in the hypoallergenic variant in another order than in said allergenic polypeptide.

According to a preferred embodiment of the present invention the at least one modified loop region comprises at least 10 amino acid residues.

The at least one loop region is preferably divided into at least two fragments, each of said fragments comprising a maximum of 20, preferably 15, more preferably 10, amino acid residues.

According to another preferred embodiment of the present invention the at least one loop region is divided into at least two fragments, each of said fragments comprising 4 to 20, preferably 5 to 15, more preferably 5 to 10, amino acid residues.

The hypoallergenic variant according to the present invention comprises disulfide bonds present also in the allergenic polypeptide.

According to a particularly preferred embodiment of the present invention the hypoallergenic variant exhibits an at least 10%, preferably an at least 20%, more preferably an at least 40%, even more preferably an at least 50%, most preferably an at least 70%, reduced IgE reactivity compared to the allergenic polypeptide from which said hypoallergenic variant has been derived.

The allergenic polypeptide is preferably an allergenic prolamin or cupin, preferably a member of the 2S albumin family, or of the allergenic bifunctional amylase/protease inhibitor family, or of the allergenic non-specific lipid transfer protein family, or of the 7S globulin (vicilin) or 11S globulin (legumin) families.

According to a preferred embodiment of the present invention the hypoallergenic variant according to the present invention can be used in the prevention, amelioration or treatment of allergic conditions, in particular food allergy.

A further aspect of the present invention relates to a nucleic acid molecule encoding a hypoallergenic variant according to the present invention.

Another aspect of the present invention relates to a vector comprising a nucleic acid molecule according to the present invention.

Another aspect of the present invention relates to a host cell comprising a nucleic acid molecule according to the present invention.

Yet another aspect of the present invention relates to the use of a hypoallergenic variant according to the present invention, a nucleic acid molecule according to the present invention, a vector according to the present invention and/or a host cell according to the present invention for the manufacture of a medicament for preventing, ameliorating or treating allergic conditions, in particular food allergy.

A further aspect of the present invention relates to a method for preventing, ameliorating or treating individuals suffering from allergic conditions, in particular food allergy, by administering to said individuals a hypoallergenic variant according to any one of the present invention, a nucleic acid molecule according to the present invention, a vector according to the present invention and/or a host cell according to the present invention.

Preferably the present invention is defined by the following embodiments:
1. Method for producing a hypoallergenic variant of an allergenic polypeptide having one or more loop regions comprising the step of modifying at least one loop region within the allergenic polypeptide by dividing said at least one loop region into at least two fragments, each of said fragments comprising at least 4 amino acid residues and combining said at least two fragments in a different order than in said allergenic polypeptide.
2. Method according to embodiment 1, wherein the at least one loop region to be modified comprises at least 10 amino acid residues.
3. Method according to embodiment 1 or 2, wherein the at least one loop region is divided into at least two fragments, each of said fragments comprising a maximum of 20, preferably 15, more preferably 10, amino acid residues.
4. Method according to embodiment 1, wherein the at least one loop region is divided into at least two fragments, each of said fragments comprising 4 to 20, preferably 5 to 15, more preferably 5 to 10, amino acid residues.
5. Method according to any one of embodiments 1 to 4, wherein the allergenic polypeptide comprises disulfide bonds.
6. Method according to any one of embodiments 1 to 5, wherein the hypoallergenic variant exhibits an at least 10%, preferably an at least 20%, more preferably an at least 40%, even more preferably an at least 50%, most preferably an at least 70%, reduced IgE reactivity compared to the allergenic polypeptide from which said hypoallergenic variant has been derived.
7. Method according to any one of embodiments 1 to 6, wherein the allergenic polypeptide is an allergenic cupin or prolamin, preferably selected from the group of allergenic prolamins or cupins, preferably a member of the 2S albumin family, or of the allergenic bifunctional amylase/protease inhibitor family, or of the allergenic non-specific lipid transfer protein family, or of the 7S globulin (vicilin) or 11S globulin (legumin) families.
8. Method according to embodiment 7, wherein the allergenic 2S albumin is derived from a plant selected from the group consisting of *Arachis hypogaea, Glycine max, Lupinus albus, Lupinus angustifolius, Corylus avellana, Juglans regia, Juglans nigra, Carya illinoinensis, Anacardium occidentale, Pistacia vera, Bertolletia excels, Fagopyrum esculentum, Fagopyrum tataricum, Sesamum indicum, Sinapis alba, Brassica juncea, Brassica napus, Brassica* rapa and *Ricinus communis.*
9. Method according to embodiment 7 or 8, wherein the allergenic 2S albumin is selected from the group consisting of Ara h 2, Ara h 6, Ara h 7, Gly m 2S albumin, Lup a delta_Conglutin, Lup an delta_Conglutin, Cor a 14, Jug r 1, Jug n 1, Car i 1, Ana o 3, Pis v 1, Ber e 1, Fag e 2, Fag t 2, Ses i 1, Ses i 2, Sin a 1, Bra j 1, Bra n 1, Bra r 1 and Ric c 1.
10. Method according to embodiment 7, wherein the allergenic 7S globulin/vicilin is derived from a plant selected from the group consisting of *Arachis hypogaea, Glycine max, Pisum sativum, Lens culinaris, Lupinus albus, Lupinus angustifolius, Corylus avellana, Juglans regia, Juglans nigra, Anacardium occidentale, Pistacia vera, Fagopyrum esculentum* and *Sesamum indicum.*
11. Method according to embodiment 7 or 10, wherein the allergenic 7S globulin/vicilin is selected from the group consisting of Ara h 1, Gly m 5, Pis s 1, Pis s 2, Len c 1, Lup a 1, Lup an 1, Cor a 11, Jug r 2, Jug n 2, Ana o 1, Pis v 3, Fag e 19kD and Ses i 3.
12. Method according to embodiment 7, wherein the allergenic 11S globulin/legumin is derived from a plant selected from the group consisting of *Arachis hypogaea, Glycine max, Lupinus albus, Lupinus angustifolius, Corylus avellana, Juglans regia, Carya illinoinensis, Anacardium occidentale, Pistacia vera, Bertolletia excels, Prunus dulcis, Fagopyrum esculentum, Fagopyrum tataricum, Sesamum indicum* and *Sinapis alba.*
13. Method according to embodiment 7 or 12, wherein the allergenic 11S globulin/legumin is selected from the group consisting of Ara h 3, Ara h 4, Gly m 6, Lup a alpha_Conglutin, Lup an alpha Conglutin, Cor a 9, Jug r 4, Car i 4, Ana o 2, Pis v 2, Pis v 5, Ber e 2, Pru du 6, Fag e 1, Fag t 1, Ses i 6, Ses i 7 and Sin a 2.
14. Method according to embodiment 7, wherein the allergenic bifunctional amylase/protease inhibitor is derived from a plant selected from the group consisting of Triticum aestivum, Hordeum vulgare and Oryza sativa.
15. Method according to embodiment 7 or 14, wherein the allergenic bifunctional amylase/protease inhibitor is selected from the group consisting of Tri a 15, Tri a 28, Tri a 29, Tri a 30, Hor v 15, BDAI-I, BTI-CMe, RAG1 and RAG2.
16. Method according to embodiment 7, wherein the allergenic non-specific lipid transfer protein is derived from a plant selected from the group consisting of *Parietaria judaica, Platanus orientalis, Ambrosia artemisifolia, Artemisia vulgaris, Helianthus annuus, Lactuca sativa, Hevea brasiliensis,* Prunus *persica, Prunus armeniaca, Prunus avium, Prunus domestica, Prunus dulcis, Malus domestica, Pyrus communis, Fragaria ananassa, Rubus idaeus, Morus nigra, Vitis vinifera, Citrus sinensis, Arachis hypogaea, Lens culinaris, Phaseolus vulgaris, Corylus avellana, Juglans regia, Triticum aesti vum, Triticum durum, Herdeum vulgare, Oryza sativa, Zea mays, Apium graveolens, Daucus carota, Brassica oleracea, Brassica rapa*, *Sinapis alba* and *Solanum lycopersicum.*
17. Method according to embodiment 7 or 16, wherein the allergenic non-specific lipid transfer protein is selected from the group consisting of Par j 1, Par j 2, Pla or 3, Amb a 6, Art v 3, Hel a 3, Lac s 1, Hev b 12, Pru p 3, Pru ar 3, Pru av 3, Pru d 3, Pru du 3, Mal d 3, Pyr c 3, Fra a 3, Rub i 3, Mor n 3, Vit v 1, Cit s 3, Ara h 9, Len c 3, Pha v 3, Cor a 8, Jug r 3, Tri a 14, Tri td 14, Hor v 14, Ory s 14, Zea m 14, Api g 2, Dau c 3, Bra o 3, Bra r 3, Sin a 3 and Lyc e 3.
18. Hypoallergenic variant of an allergenic polypeptide having one or more loop regions obtainable by a method according to any one of embodiments 1 to 17.
19. Hypoallergenic variant of an allergenic polypeptide having one or more loop regions obtainable by modifying at least one loop region within the allergenic polypeptide by dividing said at least one loop region into at least two fragments, each of said fragments comprising at least 4 amino acid residues and combining said at least two fragments in a different order than in said allergenic polypeptide.
20. Hypoallergenic variant of an allergenic polypeptide having one or more loop regions, wherein at least two fragments of at least 4 amino acid residues of at least one loop region of the allergenic polypeptide are ordered in the hypoallergenic variant in a different order than in said allergenic polypeptide.
21. Hypoallergenic variant according to embodiment 20, wherein the at least one modified loop region comprises at least 10 amino acid residues.
22. Hypoallergenic variant according to embodiment 20 or 21, wherein the at least one loop region is divided into at least two fragments, each of said fragments comprising a maximum of 20, preferably 15, more preferably 10, amino acid residues.
23. Hypoallergenic variant according to any one of embodiments 20 to 22, wherein the at least one loop region is divided into at least two fragments, each of said fragments comprising 4 to 20, preferably 5 to 15, more preferably 5 to 10, amino acid residues.
24. Hypoallergenic variant according to any one of embodiments 20 to 23, wherein the allergenic polypeptide comprises disulfide bonds.
25. Hypoallergenic variant according to any one of embodiments 20 to 24, wherein the hypoallergenic variant exhibits an at least 10%, preferably an at least 20%, more preferably an at least 40%, even more preferably an at least 50%, most preferably an at least 70%, reduced IgE reactivity compared to the allergenic polypeptide from which said hypoallergenic variant has been derived.
26. Hypoallergenic variant according to any one of embodiments 20 to 25, wherein the allergenic polypeptide is an allergenic cupin or prolamin, preferably a member of the 2S albumin family, or of the allergenic bifunctional amylase/protease inhibitor family, or of the allergenic non-specific lipid transfer protein family, or of the 7S globulin (vicilin) or 11S globulin (legumin) families.
27. Hypoallergenic variant according to any one of embodiments 18 to 26, wherein the allergenic polypeptide is a peanut allergen preferably selected from the group consisting of Ara h 1, Ara h 2, Ara h 3 and Ara h 6.
28. Hypoallergenic variant according to any one of embodiments 18 to 27, wherein the hypoallergenic variant comprises or consists of amino acid sequence SEQ ID No. 1, SEQ ID No. 2, SEQ ID No. 3, SEQ ID No. 4 or SEQ ID No. 5.
29. Hypoallergenic variant according to any one of embodiments 20 to 28 for the use in the prevention, amelioration or treatment of allergic conditions, in particular food allergy.
30. Nucleic acid molecule encoding a hypoallergenic variant according to any one of embodiments 20 to 28.
31. Vector comprising a nucleic acid molecule according to embodiment 30.
32. Host cell comprising a nucleic acid molecule according to embodiment 30 or a vector according to embodiment 31.
33. Use of a hypoallergenic variant according to any one of embodiments 20 to 28, a nucleic acid molecule according to embodiment 30, a vector according to embodiment 31 and/or a host cell according to embodiment 32 for the manufacture of a medicament for preventing, ameliorating or treating allergic conditions, in particular food allergy.
34. Method for preventing, ameliorating or treating individuals suffering from allergic conditions, in particular food allergy, by administering to said individuals a hypoallergenic variant according to any one of embodiments 20 to 28, a nucleic acid molecule according to embodiment 30, a vector according to embodiment 31 and/or a host cell according to embodiment 32.

The present invention is further illustrated by the following figures and example, however, without being restricted thereto.
Fig. 1 shows the reduced IgE binding of the modified allergens. The IgE binding from 25 individual peanut allergic patient's sera to the natural allergens and the corresponding hypoallergens was measured by OD 405 nm and is expressed as the average score for each allergen.
Fig. 2 shows RBL cell activation data induced by the hypoallergens and sera from four different peanut allergic patients.
Fig. 3 shows the amino acid sequence of mature Ara h 1 (aa 104-626, UniProt: P43238) protein and rAra h 1 mut1 construct. The swapped fragments are in bold and italic-underlined.
Fig. 4 shows the amino acid sequence of mature Ara h 2 protein (aa 22-172, UniProt: Q6PSU2), rAra h 2 mut1 and rAra h 2 mut2. The swapped fragments are in bold and italic-underlined.
Fig. 5 shows the amino acid sequence of mature Ara h 3 (aa 30-538, UniProt: Q8LKN1) protein and rAra h 3 mut1 construct. The swapped fragments are in bold and italic-underlined.
Fig. 6 shows the amino acid sequence of mature Ara h 6 protein (aa 22-145, UniProt: Q647G9) and rAra h 6 mut1. The swapped fragments are in bold and italic-underlined.

### EXAMPLE:

### Material and Methods:

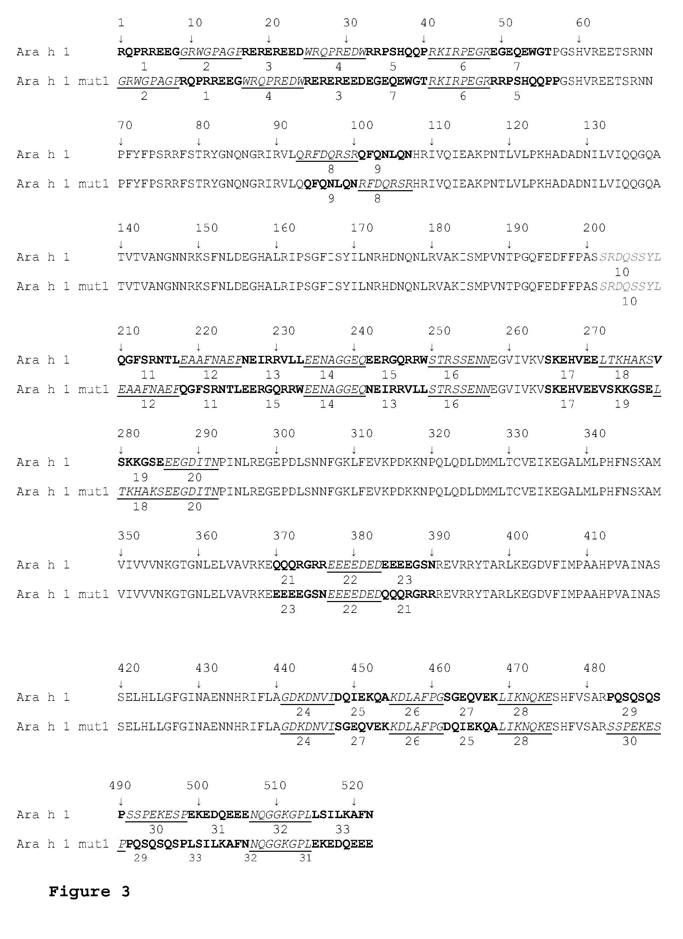

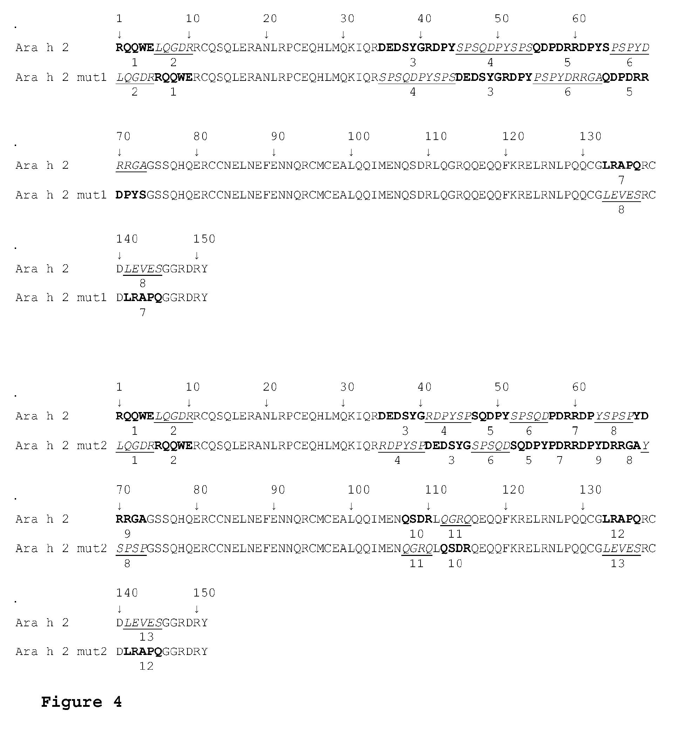

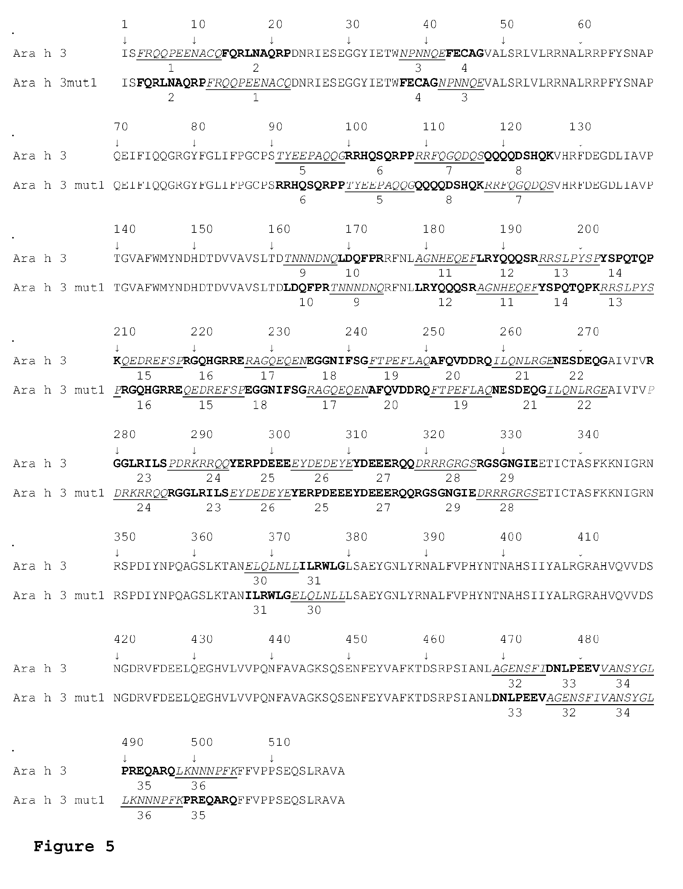

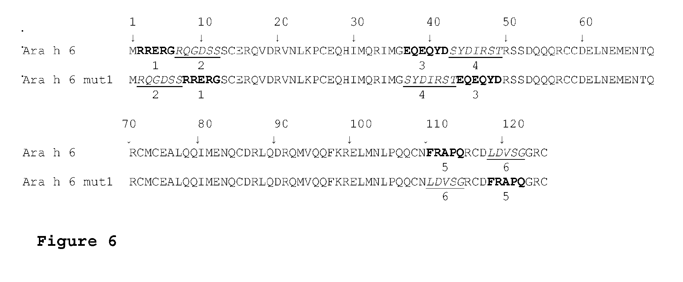

Based on an in depth in silico analysis of the allergen structure and following the principle of this invention, hypoallergens of the major peanut allergens, Ara h 1, Ara h 2, Ara h 3 and Ara h 6 were designed.
For the expression of hypoallergenic rAra h 1 mut1, rAra h 2 mut1, rAra h 2 mut2, rAra h 3 mut1 and rAra h 6 mut1 molecules, the coding DNA sequence of mature Ara h 1 (aa 104-626, UniProt: P43238), Ara h 2 (aa 22-172, UniProt: Q6PSU2), Ara h 3 (aa 30-538, UniProt: Q8LKN1),and Ara h 6 (aa 22-145, UniProt: Q647G9) were used as templates. The swapped fragments are shown in SEQ ID No.1-5 and Fig. 3 to 6.

For DNA sequence construction of hypoallergenic Ara h 1 and Ara h 3, changes were made in the N- (aa 1-56 of Ara h 1 and aa 3-22 of Ara h 3) and C- termini (aa 441-522 of Ara h 1 and aa 469-505 of Ara h 3) and the regions of the three loops (sequence positions of Ara h 1: 94-107, 202-291, 368-388 and sequence position of Ara h 3: 23-42, 57-67, 111-146, 182-194, 199-293, 299-354, 387-399, 489-524). The hypoallergens were designed by swapping neighbouring peptide sequences within the mature Ara h 1 (UniProt: P43238) and Ara h 3 (PDB: 3c3v; UniProt: Q8LKN1). The mutated allergens were designated rAra h 1 mut 1 and rAra h 3 mut1. Mutants of Ara h 2 and Ara h 6 were constructed by using the three-dimensional structure of Ara h 6, a 2S albumin from peanut (PDBID: 1W2Q) and the amino sequence of its homologue, Ara h 2 (UniProt: Q6PSU2). The changes were made to the sequences of the N- (aa 1-10 of Ara h 2 and aa 2-12 of Arah 6) and C-termini (aa 131-145 of Ara h 2 and aa 111-123 of Ara h 6) and in the large loop (sequence positions of Ara h 2: 35-74 and sequence positions of Ara h 6: 35-48). A second construct of Ara h 2 was designed by additional changes in the small loop (sequence position of Ara h 2: 83-91). These modified molecules were designated as rAra h 2 mut1, rAra h 2 mut2 and rAra h 6 mut1.

### Results:

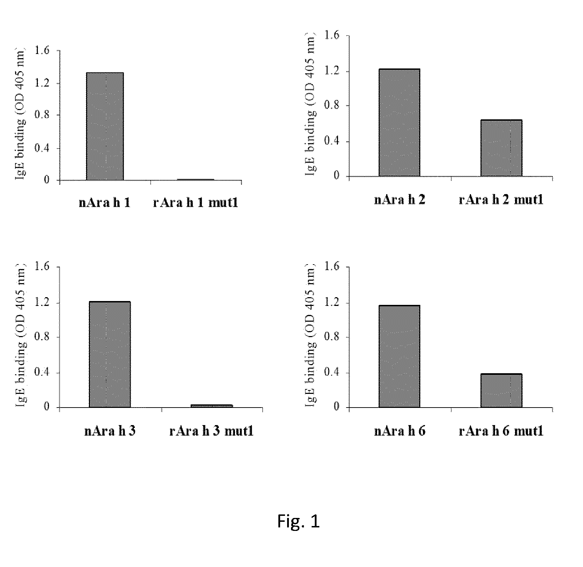

The ability of the hypoallergens to bind IgE was tested by ELISA using 25 sera from peanut allergic patients (Fig. 1). The results showed that the modified versions of the allergens bound significantly less IgE than their natural counterparts, 23 (92%) of 25 sera recognized natural Ara h 1 (nAra h 1). Seventeen of the twenty three sera (74%) only showed extremely low IgE-binding of 1.5-6% to rAra h 1 mut1 in comparison with IgE-binding to nAra h 1. Six sera (26%) showed no IgE-binding at all. Similar results were obtained for Ara h 3 mut1. Twenty two sera (88%) recognized the natural allergen. Sixteen sera (64%) demonstrated a reduction of IgE-binding of about 95% to the hypoallergen rAra h 3 mut1, and the rest showed no IgE binding activity. Ara h 2 mut1 and Ara h 6 mut1 revealed 50% or less IgE reactivity, when compared with IgE-binding to their natural counterparts.

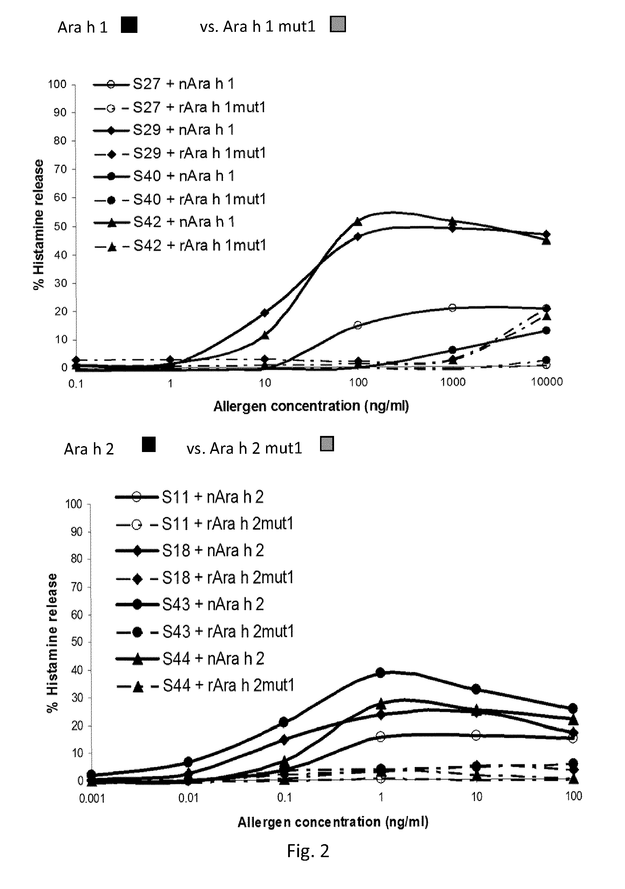

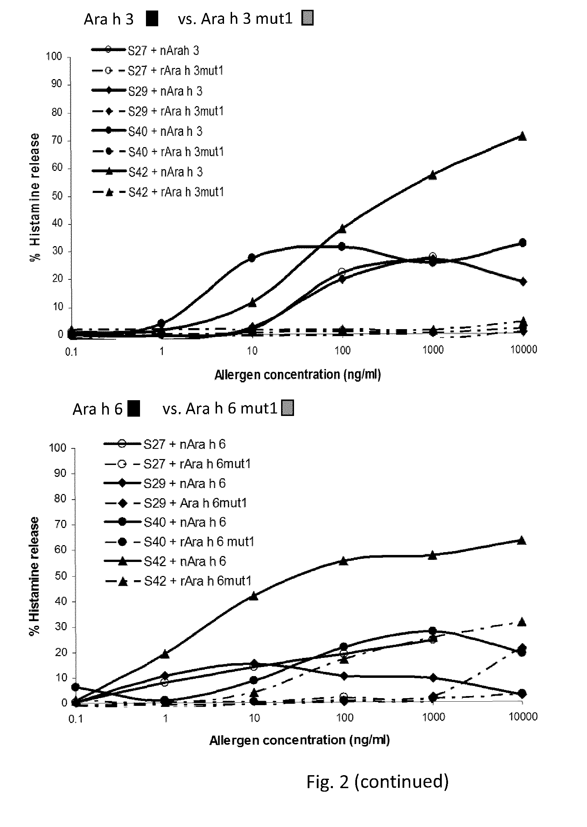

Furthermore, the IgE cross-linking capacities of the mutated allergens (Fig. 2) were analysed. Rat basophilic leukemia (RBL) cells were sensitized with IgE of four sera from peanut allergic patients and challenged by serial dilutions of the allergens. The antigen-specific release was quantified by measuring β-hexosaminidase activity. For patients' sera S29 and S42, nAra h 1 showed the highest degranulating activity with a maximum release of about 55% obtained at an allergen concentration of 100 ng/ml. For the same sera, rAra h 1 mut1 revealed the highest degranulating activity with a maximum release of about 20% obtained at 10,000 ng/ml hypoallergen. In this case, nAra h 1 showed 36% more degranulating activity at a concentration two orders of magnitude higher than that of rAra h 1 mut1. Natural Ara h 1 induced a maximum mediator release of about 20% and 15% at a concentration of 1,000 ng/ml and 10,000 ng/ml by using serum S27 and serum S40, respectively. In contrast, the hypoallergen rAra h 1 mut1 showed a reduced degranulation activity (only 5%) using the same sera. Ara h 2 mut1 and Ara h 3 showed no degranulation activity and release curves of the mutated Ara h 6 mut1 are also clearly shifted to the right compared to the release curve of natural Ara h 6.

## Claims

1. Method for producing a hypoallergenic variant of an allergenic polypeptide having one or more loop regions comprising the step of modifying at least one loop region within the allergenic polypeptide by dividing said at least one loop region into at least two fragments, each of said fragments comprising at least 4 amino acid residues and combining said at least two fragments in a different order than in said allergenic polypeptide.

2. Method according to claim 1, wherein the at least one loop region to be modified comprises at least 10 amino acid residues.

3. Method according to claim 1 or 2, wherein the at least one loop region is divided into at least two fragments, each of said fragments comprising a maximum of 20, preferably 15, more preferably 10, amino acid residues.

4. Method according to claim 1, wherein the at least one loop region is divided into at least two fragments, each of said fragments comprising 4 to 20, preferably 5 to 15, more preferably 5 to 10, amino acid residues.

5. Method according to any one of claims 1 to 4, wherein the allergenic polypeptide is an allergenic cupin or prolamin, preferably selected from the group of allergenic prolamins or cupins, preferably a member of the 2S albumin family, or of the allergenic bifunctional amylase/protease inhibitor family, or of the allergenic non-specific lipid transfer protein family, or of the 7S globulin (vicilin) or 11S globulin (legumin) families.

6. Hypoallergenic variant of an allergenic polypeptide having one or more loop regions obtainable by a method according to any one of claims 1 to 5.

7. Hypoallergenic variant of an allergenic polypeptide having one or more loop regions obtainable by modifying at least one loop region within the allergenic polypeptide by dividing said at least one loop region into at least two fragments, each of said fragments comprising at least 4 amino acid residues and combining said at least two fragments in a different order than in said allergenic polypeptide.

8. Hypoallergenic variant of an allergenic polypeptide having one or more loop regions, wherein at least two fragments of at least 4 amino acid residues of at least one loop region of the allergenic polypeptide are ordered in the hypoallergenic variant in another order than in said allergenic polypeptide.

9. Hypoallergenic variant according to claim 8, wherein the at least one modified loop region comprises at least 10 amino acid residues.

10. Hypoallergenic variant according to claim 8 or 9, wherein the at least one loop region is divided into at least two fragments, each of said fragments comprising a maximum of 20, preferably 15, more preferably 10, amino acid residues.

11. Hypoallergenic variant according to any one of claims 8 to 10, wherein the at least one loop region is divided into at least two fragments, each of said fragments comprising 4 to 20, preferably 5 to 15, more preferably 5 to 10, amino acid residues.

12. Hypoallergenic variant according to any one of claims 6 to 10, wherein the allergenic polypeptide is a peanut allergen preferably selected from the group consisting of Ara h 1, Ara h 2, Ara h 3 and Ara h 6.

13. Hypoallergenic variant according to any one of claims 6 to 12, wherein the hypoallergenic variant comprises or consists of amino acid sequence SEQ ID No. 1, SEQ ID No. 2, SEQ ID No. 3, SEQ ID No. 4 or SEQ ID No. 5.

14. Hypoallergenic variant according to any one of claims 8 to 13 for the use in the prevention, amelioration or treatment of allergic conditions, in particular food allergy.

15. Nucleic acid molecule encoding a hypoallergenic variant according to any one of claims 8 to 13.

16. Vector comprising a nucleic acid molecule according to claim 15.

17. Host cell comprising a nucleic acid molecule according to claim 15 or a vector according to claim 16.

18. Use of a hypoallergenic variant according to any one of claims 8 to 13, a nucleic acid molecule according to claim 15, a vector according to claim 16 and/or a host cell according to claim 17 for the manufacture of a medicament for preventing, ameliorating or treating allergic conditions, in particular food allergy.
